# EUROPEAN PATENT SPECIFICATION

(11) **EP 0 724 479 B1**
(45) Date of publication and mention of the grant of the patent: **13.06.2001**
(21) Application number: 94926216.6
(22) Date of filing: 22.08.1994
(51) Int. Cl.: B01J 23/66, C07D 301/10

(54) **ETHYLENE OXIDE CATALYST**
ETHYLENOXIDKATALYSATOR
CATALYSEUR D'OXYDE D'ETHYLENE

(30) Priority: 23.08.1993 US 110282
(43) Date of publication of application: 07.08.1996
(73) Proprietor: SHELL INTERNATIONALE RESEARCH MAATSCHAPPIJ B.V., 2596 HR Den Haag (NL)
(72) Inventor: MATUSZ, Marek, Houston, TX 77084 (US)
(86) International application number: EP9402783
(87) International publication number: WO9505896

(56) References cited:
- EP-A- 0 059 422
- EP-A- 0 172 565
- EP-A- 0 247 414
- US-A- 4 908 343
- DATABASE WPI Week 8329, Derwent Publications Ltd., London, GB; AN 83-714123 & JP,A,58 098 144 (MITSUBISCHI PETROCHEMICAL KK) 10 June 1983
- DATABASE WPI Week 9230, Derwent Publications Ltd., London, GB; AN 92-248428 & SU,A,1 685 510 (V. A. DAVYDOV ET AL.) 23 October 1991

## Description

The invention relates to silver-containing catalysts suitable for the preparation of ethylene oxide.

Catalysts for the production of ethylene oxide from ethylene and molecular oxygen are generally supported silver catalysts. Such catalysts are typically promoted with alkali metals. The use of small amounts of the alkali metals potassium, rubidium and cesium were noted as useful promoters in supported silver catalysts in U.S. Patent No. 3,962,136, issued June 8, 1976, and U.S. Patent No. 4,010,115, issued March 1, 1977. The use of other co-promoters, such as rhenium, or rhenium along with sulphur, molybdenum, tungsten and chromium is disclosed in U.S. Patent No. 4,766,105, issued August 23, 1988, and U.S. Patent No. 4,808,738, issued February 28, 1989. U.S. Patent No. 4,908,343, issued March 13, 1990, discloses a supported silver catalyst containing a mixture of a cesium salt and one or more alkali metal and alkaline earth metal salts.

The use of chloride moderators to enhance the yield of silverbased ethylene oxide catalysts is known in the art. The chloride is provided as chlorohydrocarbon moderators in the reaction gases or as chloride anions incorporated into the catalyst during the catalyst preparation phase. Earlier practice tended to emphasize the latter practice whereas more recent practice tends to emphasize the former practice. The use of chloride-containing compounds to prepare catalysts can be found in U.S. Patent Numbers 2,615,900, issued October 28, 1952; 2,709,173, issued May 24, 1955; 2,765,283, issued October 2, 1956; 2,769,016, issued October 30, 1956; 2,799,687, issued July 16, 1957; 3,585,217, issued June 15 1971; 4,415,476, issued November 15, 1983; and 4,904,343, issued March 13, 1990.

In European Patent application No. 0 172 565, published 26 February 1986, there is disclosed a catalyst for the production of ethylene oxide, comprising at least silver grains deposited on a porous carrier, the grains having an average diameter of 0.05 to 0.4 micrometer and a concentration in the innermost of the carrier of at least 65% of their concentration on the outside surface of the carrier, which is achieved by heating the carrier with superheated steam to deposit the silver in it. This catalyst composition can further comprise a cationic component such as an alkali metal, the balancing anion possibly being a halogen such as fluorine, chlorine and bromine, in an amount of from 5 ppm to 0.1% by weight.

During the initial phase of catalyst start-up of the alkali metal doped silver-based catalysts in commercial ethylene oxide reactors even in the presence of chlorohydrocarbon moderators the catalysts experience the so-called breakthrough phase, during which the oxygen conversion is very high and the selectivity is very low. The reaction is difficult to control at this stage. It might take days in a commercial start-up for the conversion to drop so that the reaction can be controlled. Substantial economic incentive exists to shorten the start-up period and make the catalysts operate at higher selectivities since the selectivity determines the yield of ethylene oxide product.

In U.S. patent numbers 5,155,242, issued October 13, 1992, and 4,874,879, issued October 17, 1989, there is disclosed an improved start-up procedure wherein the catalysts are subjected to a presoak period in the presence of a chlorohydrocarbon moderator at a temperature less than the operating temperature of the reactor. While this technique does improve catalyst start-up, it can be subject to operator error. Over- and under-moderating with the chlorohydrocarbon moderator during the presoak can adversely affect start-up and post start-up operations. It would be desirable to have a catalyst that could be loaded into a reactor and started up in the presence of a chlorohydrocarbon moderator without any special pre-chloriding procedures needing to be applied to the catalyst in the reactor.

The invention relates to a catalyst for the vapour phase production of ethylene oxide from ethylene and oxygen in the presence of a hydrocarbon moderator comprising, expressed as the metal by weight of the total catalyst, from 12 to 40 percent of silver and from 10 to 3000 parts per million of an alkali metal at least 50% of which is selected from potassium, rubidium, cesium, lithium and mixtures thereof, and further comprising prior to the first use of the catalyst from 0.4x10⁻³ to 4x10⁻³ moles of chloride per mole of silver, deposited by impregnation followed by curing without the use of superheated steam on a carrier comprising at least 85 percent by weight of alpha alumina.

The invention also relates to a process for preparing a catalyst for the vapour phase production of ethylene oxide from ethylene and molecular oxygen which comprises impregnating a carrier comprising at least 85 percent by weight of alpha-alumina with one or more solutions comprising solvent having silver compound(s) dissolved therein, compound(s) of alkali metal(s) selected from potassium, rubidium, cesium, lithium and mixtures thereof dissolved therein and chloride-containing compounds dissolved therein sufficient to deposit on the support from 12 to 40 percent by weight of the total catalyst of the silver compound(s), expressed as the metal, from 10 to 3000 parts per million by weight of the alkali metal compound(s), expressed as the metal, by weight of the total catalyst and from 0.4x10⁻³ to 4x10⁻³ moles of chloride per mole of silver deposited and curing without the use of superheated steam.

It has been found that catalysts containing a selected amount of chloride have improved start-up characteristics over catalysts without such chloride when such catalysts are started up in the presence of ethylene, oxygen and a chlorohydrocarbon moderator. The presence of the chloride allows the catalyst to reach higher selectivity levels earlier than catalysts without such chloride. Thus, an improved process for starting up a fixed bed ethylene oxide reactor containing fresh or unused catalyst is obtained.

Figure 1 is a plot of the selectivity of ethylene to ethylene oxide as a function of time for a Comparative Catalyst A and two catalysts of this invention (B and C) during the start-up of an ethylene oxide reactor.

Figure 2 is a plot of the oxygen conversion as a function of time for a Comparative Catalyst A and two catalysts of this invention (B and C) during the start-up of an ethylene oxide reactor.

Figure 3 is a plot of the selectivity of ethylene to ethylene oxide as a function of time for a Comparative Catalyst A and a catalyst of this invention (E) during the start-up of an ethylene oxide reactor.

Figure 4 is a plot of the oxygen conversion as a function of time for a Comparative Catalyst A and a catalyst of this invention (E) during the start-up of an ethylene oxide reactor.

The catalysts of the instant invention comprise a catalytically effective amount of silver, a promoting amount of alkali metal and, prior to the catalyst's first use in producing ethylene oxide, from 0.4x10⁻³ to 4x10⁻³ moles of chloride per mole of silver supported on an alpha alumina support. Other promoters in promoting amounts may be optionally present such as rare earths, magnesium, rhenium and rhenium co-promoters selected from sulphur, chromium, molybdenum, tungsten and mixtures thereof.

In general, the catalysts of the present invention are prepared by impregnating porous refractory supports comprising alpha alumina with silver ions or compound(s), complex(es) and/or salt(s) dissolved in a suitable solvent sufficient to cause deposition on the support of from 1 to 40, preferably from 1 to 30 percent by weight, basis the weight of the total catalyst, of silver. The impregnated support is then separated from the solution and the deposited silver compound is reduced to metallic silver. Also deposited on the support either prior to, coincidentally with, or subsequent to the deposition of the silver will be suitable ions, or compound(s) and/or salt(s) of alkali metal dissolved in a suitable solvent. Also deposited on the carrier coincidentally with the deposition of the silver and/or alkali metal will be suitable optional promoter compound(s), complex(es) and/or salt(s) dissolved in an appropriate solvent. Also deposited on the support either prior to, coincidentally with, or subsequent to the deposition of the silver and/or alkali metal and/or optional promoters will be chlorine in the form of chloride ("Cl⁻¹") dissolved in an appropriate solvent.

The carrier or support employed in these catalysts in its broadest aspects can be any of the large number of conventional, porous refractory catalyst carriers or support materials which are considered relatively inert in the presence of ethylene oxidation feeds, products and reaction conditions. Such conventional materials are known to those skilled in the art and may be of natural or synthetic origin and preferably are of a macroporous structure, i.e., a structure having a surface area below 10 m²/g and preferably below 3 m²/g. Particularly suitable supports are those of aluminous composition. Preferred supports comprise the aluminous materials, in particular those comprising alpha alumina. In the case of alpha alumina-containing supports, preference is given to those having a specific surface area as measured by the B.E.T. method of from 0.03 m²/g to 10 m²/g, preferably from 0.05 m²/g to 5 m²/g, more preferably from 0.1 m²/g to 3 m²/g, and a water pore volume as measured by conventional water absorption techniques of from 0.1 to 0.75 ml/g by volume. The B.E.T. method for determining specific surface area is described in detail in Brunauer, S., Emmet, P. Y. and Teller, E., J. Am. Chem. Soc., 60, 309-16 (1938).

Certain types of alpha alumina containing supports are particularly preferred. These alpha alumina supports have relatively uniform pore diameters and are more fully characterized by having B.E.T. specific surface areas of from 0.1 m²/g to 3 m²/g, preferably from 0.1 m²/g to 2 m²/g, and water pore volumes of from 0.10 ml/g to 0.55 ml/g. Typical properties of some supports found particularly useful in the present invention are presented in Table I. Suitable manufacturers of carriers comparable to those in Table I include Norton Company and United Catalysts, Inc. (UCI).

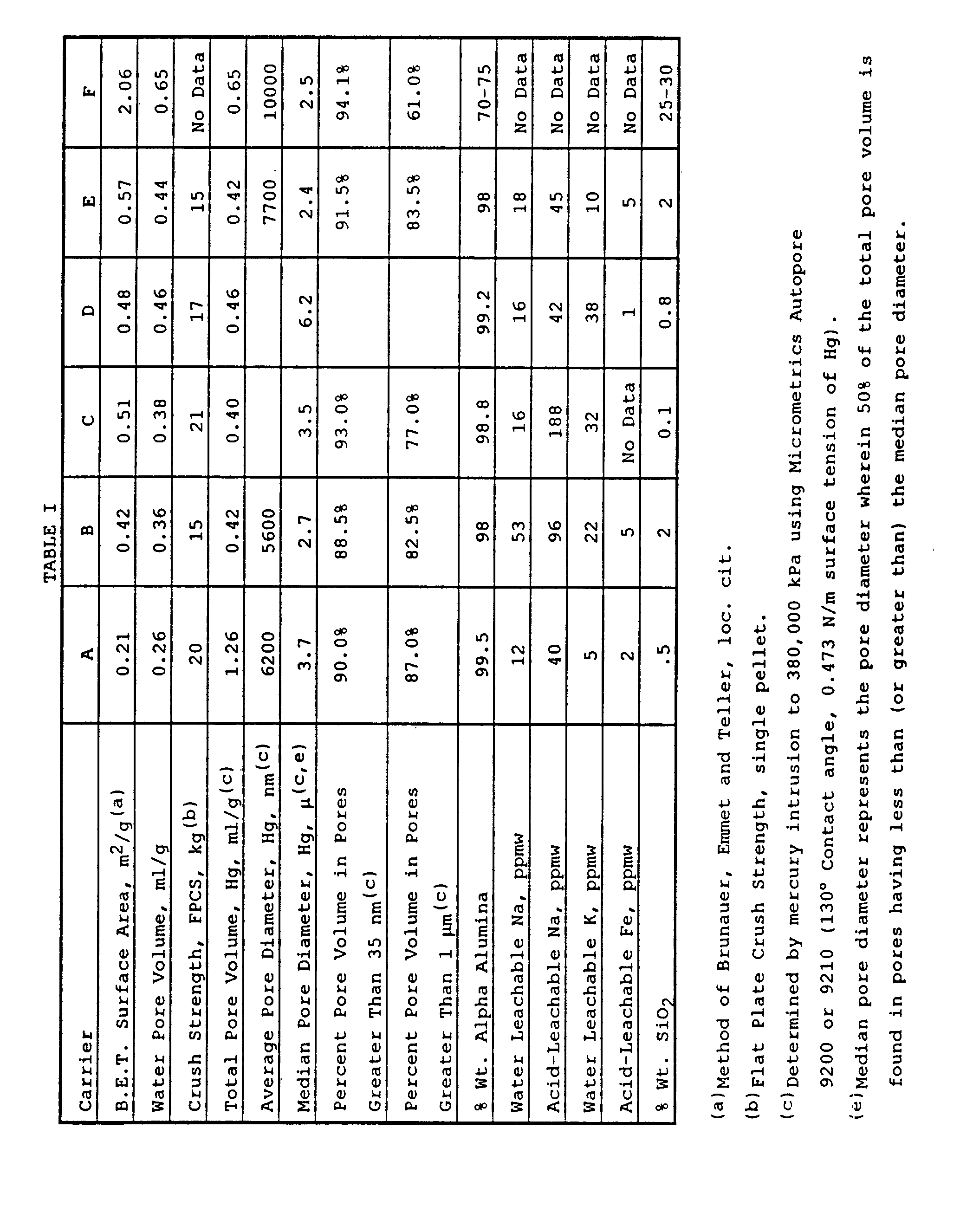

Of the carriers listed in TABLE I D is a preferred carrier. Other desirable supports are those such as described in U.S. patent No. 5,145,824.

The support, irrespective of the character of the support or carrier used, is preferably shaped into particles, chunks, pieces, pellets, rings, spheres, wagon wheels, and the like of a size suitable for use in fixed bed reactors. Conventional commercial fixed bed reactors are typically in the form of a plurality of parallel elongated tubes (in a suitable shell) approximately 18 to 69 mm O.D. and 12 to 25 mm I.D. and 4.5-14 m long filled with catalyst. In such reactors, it is desirable to use a support formed into a rounded shape, such as, for example, spheres, pellets, rings, tablets and the like, having diameters from about 2.5 to 20 mm.

Particular supports having differing properties such as surface area and pore volume may be selected in order to provide particular catalytic properties. With regard to surface area (B.E.T.), a possible lower limit is 0.01 m²/g and a possible upper limit is 10 m²/g. With regard to water pore volume, a possible lower limit is 0.05 ml/g and a possible upper limit is 0.8 ml/g.

The catalysts of the present invention are prepared by a technique in which the alkali metal promoters and the chloride in the form of soluble salts and/or compounds are deposited on the catalyst and/or support prior to, simultaneously with, or subsequent to the deposition of the silver and each other. The preferred method is to deposit silver, alkali metal and chloride simultaneously on the support, that is, in a single impregnation step, although it is believed that the individual or concurrent deposition of the alkali metal and chloride prior to and/or subsequent to the deposition of the silver would also produce suitable catalysts.

Promoting amounts of alkali metal or mixtures of alkali metal are deposited on a porous support using a suitable solution. Although alkali metals exist in a pure metallic state, they are not suitable for use in that form. They are used as ions or compounds of alkali metals dissolved in a suitable solvent for impregnation purposes. The carrier is impregnated with a solution of alkali metal promoter ions, salt(s) and/or compound(s) before, during or after impregnation of the silver ions or salt(s), complex(es), and/or compound(s) has taken place. An alkali metal promoter may even be deposited on the carrier after reduction to metallic silver has taken place. The promoting amount of alkali metal utilized will depend on several variables, such as, for example, the surface area and pore structure and surface chemical properties of the carrier used, the silver content of the catalyst and the particular ions used in conjunction with the alkali metal cation, optional co-promoters. The amount of alkali metal promoter deposited upon the support or present on the catalyst generally lies between about 10 parts per million and about 3000 parts per million, preferably between about 15 parts per million and about 2000 parts per million and more preferably, between about 20 parts per million and about 1500 parts per million by weight of total catalyst. Most preferably, the amount ranges between about 50 parts per million and about 1000 parts per million by weight of the total catalyst. The degree of benefit obtained within the above-defined limits will vary depending upon particular properties and characteristics, such as, for example, reaction conditions, catalyst preparative techniques, surface area and pore structure and surface chemical properties of the carrier utilized, silver content of the catalyst, and other compounds, cations or anions present in addition to alkali metal ions , and the above-defined limits were selected to cover the widest possible variations in properties and characteristics. The effects of these variation in properties are readily determined by experimentation. The alkali metal promoters are present on the catalysts in the form of cations (ions) or compounds of complexes or surface compounds or surface complexes rather than as the extremely active free alkali metals, although for convenience purposes in this specification and claims they are referred to as "alkali metal" or "alkali metal promoters" even though they are not present on the catalyst as metallic elements. For purposes of convenience, the amount of alkali metal deposited on the support or present on the catalyst is expressed as the metal. Without intending to limit the scope of the invention, it is believed that the alkali metal compounds are oxidic compounds. More particularly, it is believed that the alkali metal compounds are probably in the form of mixed surface oxides or double surface oxides or complex surface oxides with the aluminium of the support and/or the silver of the catalyst, possibly in combination with species contained in or formed from the reaction mixture, such as, for example, chlorides or carbonates or residual species from the impregnating solution(s).

According to the invention at least a major proportion (greater than 50%) of the alkali metals are selected from the group consisting of potassium, rubidium, cesium, and mixtures thereof. As used herein, the term "alkali metal" and cognates thereof refers to the alkali metals selected from the group consisting of lithium, sodium, potassium, rubidium, cesium and mixtures thereof. As used herein, the term "mixtures of alkali metals" or cognates of these terms refers to the use of two or more of the alkali metals, as appropriate, to provide a promoting effect. Non-limiting examples include cesium plus rubidium, cesium plus potassium, cesium plus sodium, cesium plus lithium, cesium plus rubidium plus sodium, cesium plus potassium plus sodium, cesium plus lithium plus sodium, cesium plus rubidium plus potassium plus sodium, cesium plus rubidium plus potassium plus lithium, cesium plus potassium plus lithium and the like. A preferred alkali metal promoter is cesium. A particularly preferred alkali metal promoter is cesium plus at least one additional alkali metal. The additional alkali metal is preferably selected from sodium, lithium and mixtures thereof, with lithium being preferred.

It should be understood that the amounts of alkali metal promoters on the catalysts are not necessarily the total amounts of these metals present in the catalyst. Rather, they are the amounts of alkali metal promoters which have been added to the catalyst by impregnation with a suitable solution of ions, salts and/or compounds and/or complexes of alkali metals. These amounts do not include amounts of alkali metals which are locked into the support, for example, by calcining, or are not extractable in a suitable solvent such as water or lower alkanol or amine or mixtures thereof and do not provide a promoting effect. It is also understood that a source of the alkali metal promoter ions, salts and/or compounds used to promote the catalyst may be the carrier. That is, the carrier may contain extractable amounts of alkali metal that can be extracted with a suitable solvent, such as water or lower alkanol, thus preparing an impregnating solution from which the alkali metal ions, salts and/or compounds are deposited or redeposited on the support.

Other promoters and copromoters can be used in conjunction with the silver and alkali metal promoters. Non-limiting examples of other promoters include rhenium, sulphur, molybdenum, tungsten and chromium (see U.S. patent no. 4,766,105, issued August 23, 1988); sulfate anion, fluoride anion, oxyanions of Groups 3b to 6b (see U.S. patent no. 5,102,848, issued April 7, 1992); (i) oxyanions of an element selected from Groups 3 through 7b and (ii) alkali(ne) metal salts with anions of halides, and oxyanions selected from Groups 3a to 7a and 3b through 7b (see U.S. patent no. 4,908,343, issued March 13, 1990).

Moderating amounts of chloride are deposited on a porous support using a suitable impregnating solution. It is understood that chloride when deposited on the support will be present in the anionic state, i.e., as Cl⁻¹, and that there will also be present on the support a balancing cation. The exact form of this balancing cation is not known, although it is speculated that will be one of the promoter cations or silver or both. The chloride is deposited as ions, salts or compounds dissolved in a suitable solvent for impregnation purposes. The carrier is impregnated with a solution of the chloride moderator ions, salt(s) and/or compound(s) before, during or after impregnation of the silver ions or salt(s), complex(es), and/or compound(s) has taken place and before, during or after impregnation of the promoter ions or salt(s), complex(es), and/or compound(s) has taken place. The chloride moderator may even be deposited on the carrier after reduction to metallic silver has taken place. Suitable chloride-containing salts used to prepare the impregnating solutions include promoter chlorides such as lithium chloride, sodium chloride, potassium chloride, rubidium chloride and cesium chloride as well as ammonium chloride. Ammonium chloride is a preferred salt for use in preparing the chloride-containing impregnating solutions. Other compounds which decompose to the chloride ion upon processing of the catalyst are also suitable. The chloride-containing impregnating solutions will normally contain at least a small amount of water to enhance solubility of the chloride-containing salt or compound.

The amount of chloride deposited upon the support or present on the catalyst is a function of the amount of silver present on the catalyst and generally ranges from about 0.4x10⁻³ to about 4x10⁻³ moles of chloride per mole of silver. Lesser amounts do not provide a moderating effect and greater amounts adversely effect activity and selectivity. In a preferred embodiment where the catalyst contains from about 12 to about 18 weight percent of silver, the chloride content will range from about 20 parts per million to about 200 parts per million by weight of the total catalyst.

The term "moderating" as used herein with reference to the chloride deposited on the fresh or unused catalyst refers to a change in the catalyst properties that allows the catalyst during start-up to obtain higher selectivities in a shorter period of time than does a catalyst that has not been moderated. It is also understood that the moderating amounts of chloride deposited on the support are those amounts that are applied to a fresh or unused catalyst by an impregnation step. During use a silver-based ethylene oxide catalyst will obtain a certain amount of chloride deposited thereon because the catalyst is operated in an environment containing small amounts of chlorohydrocarbons, but this latter amount of chloride is not considered as part of the moderating amount of chloride referred to herein as originally deposited on the support.

Generally, the carrier is contacted with a silver salt, a silver compound, or a silver complex which has been dissolved in an aqueous solution, so that the carrier is impregnated with said aqueous solution; thereafter the impregnated carrier is separated form the aqueous solution, e.g., by centrifugation or filtration and then dried. The thus obtained impregnated carrier is heated to reduce the silver to metallic silver. It is conveniently heated to a temperature in the range of from 50°C to 600°C, during a period sufficient to cause reduction of the silver salt, compound or complex to metallic silver and to form a layer of finely divided silver, which is bound to the surface of the carrier, both the exterior and pore surface. Air, or other oxidizing gas, reducing gas, an inert gas or mixtures thereof may be conducted over the carrier during this heating step.

There are several known methods to add the silver to the carrier or support. The carrier may be impregnated with an aqueous solution containing silver nitrate dissolved therein, and then dried, after which drying step the silver nitrate is reduced with hydrogen or hydrazine. The carrier may also be impregnated with an ammoniacal solution of silver oxalate or silver carbonate, and then dried, after which drying step the silver oxalate or silver carbonate is reduced to metallic silver by heating, e.g., to 600°C. Specific solutions of silver salts with solubilizing and reducing agents may be employed as well, e.g., combinations of the vicinal alkanolamines, alkyldiamines and ammonia. One such example of a solution of silver salts comprises an impregnating solution comprising a silver salt of a carboxylic acid, an organic amine alkaline solubilizing/reducing agent, and an aqueous solvent.

These solubilizing/reducing agents are generally added in the amount of from about 0.1 to about 10 moles per mole of silver present.

One method of preparing the silver containing catalyst can be found in U.S. Patent 3,702,259. Other methods for preparing the silver-containing catalysts which in addition contain higher alkali metal promoters can be found in U.S. Patent 4,010,115; and U.S. Patent 4,356,312; U.S. Patent 3,962,136 and U.S. Patent 4,012,425, 1977. Methods for preparing silver-containing catalysts containing higher alkali metal and rhenium promoters can be found in U.S. Patent No. 4,761,394, and methods for silver-containing catalysts containing higher alkali metal and rhenium promoters and rhenium co-promoters can be found in U.S. Patent No. 4,766,105. Methods for preparing silver-containing catalysts with a variety of different promoters are found in U.S. patents 4,908,343 and 5,057,481.

The concentration of the silver (expressed as the metal) in the silver-containing solution will range from 1 g/l up to the solubility limit when a single impregnation is utilized. The concentration of the alkali metal (expressed as the metal) will range from 1 x 10⁻³ g/l up to 12 g/l and preferably, from 10 x 10³ g/l to 12 g/l when a single impregnation step is utilized. The concentration of the chloride will range from 1 x 10⁻⁴ g/l up to 1 g/l and preferably, from 5 x 10⁻⁴ g/l to 0.1 g/l when a single impregnation step is utilized. Concentrations selected within the above noted ranges will depend upon the pore volume of the catalyst, the final amount desired in the final catalyst and whether the impregnation is single or multiple.

The silver catalysts according to the present invention have been shown to have unique start-up characteristics in an ethylene oxidation process. The conditions for carrying out such an oxidation reaction in the presence of the silver catalysts according to the present invention broadly comprise those already described in the prior art. This applies, for example, to suitable temperatures, pressures, residence times, diluent materials such as nitrogen, carbon dioxide, steam, argon, methane or other saturated hydrocarbons, to the presence of moderating agents to control the catalytic action, for example, 1-2-dichloroethane, vinyl chloride, ethyl chloride or chlorinated polyphenyl compounds, to the desirability of employing recycle operations or applying successive conversations in different reactors to increase the yields of ethylene oxide, and to any other special conditions which may be selected in processes for preparing ethylene oxide. Pressures in the range of from atmospheric to about 3500 kPa are generally employed. Higher pressures, however, are not excluded. Molecular oxygen employed as reactant can be obtained from conventional sources. The suitable oxygen charge may consist essentially or relatively pure oxygen, a concentrated oxygen stream comprising oxygen in major amount with lesser amounts of one or more diluents, such as nitrogen and argon, or another oxygen-containing stream, such as air. It is therefore evident that the use of the present silver catalysts in ethylene oxide reactions is in no way limited to the use of specific conditions among those which are known to be effective. For purposes of illustration only, the following table shows the range of conditions that are often used in current commercial ethylene oxide reactor units.

**TABLE II**

| | |
|---|---|
| *GHSV | 1500-10,000 |
| Inlet Pressure | 150-400 psig |

| Inlet Feed | |
|---|---|
| Ethylene | 1-40% |
| O₂ | 3-12% |
| Ethane | 0-3% |
| Chlorohydrocarbon moderator Argon and/or methane and/or nitrogen diluent | 0.3-50 ppmv total Balance |
| Coolant temperature | 180-315 °C |
| Catalyst temperature | 180-325 °C |
| O₂ conversion level | 10-60% |
| EO Production (Work Rate) | 32-320 kg EO/m³ |
| | catalyst/hr. |

| | |
|---|---|
| *Volume units of gas at standard temperature and pressure passing over one volume unit of packed catalyst per hour. | |

In general terms an ethylene oxide reactor containing fresh or unused catalyst is started up by first heating the reactor to about 5°C- 50°C below the normal reaction temperature and passing nitrogen over the catalyst. The high temperature converts a significant portion of the organic nitrogen compounds used in the manufacture of the catalyst to nitrogen-containing gases which are swept up in the nitrogen stream and removed from the reactor. The nitrogen purge may be omitted if the impurities on the catalyst are low. The nitrogen flow, if utilized, is passed over the catalyst at a flow rate typically between 5 to 40%, preferably between 15 and 25% of the design flow rate. The nitrogen flow may be initiated before reactor heatup, during reactor heatup or after the reactor has reached the desired temperature. The nitrogen gas is typically passed over the catalyst for a period of time ranging from 1/2 of a day to 7 days. During this purge time the nitrogen stream is monitored for nitrogen-containing decomposition products from the catalyst. When nitrogen is not used, the reactor may be pressurized with ethylene, methane or other non-oxidizing gas.

After the nitrogen-containing decomposition products have been removed to a suitable low level, generally less than 10 ppm, the recycle loop to the ethylene oxide reactor is then pressurized with ethylene and a suitable ballast gas such as methane in preparation for a start-up. A gas flow rate of between 5 to 40% of design rate, preferably from 15 to 25% of design rate is maintained over the reactor.

A chlorohydrocarbon moderator is then added to the recycle gas stream being fed to the ethylene oxide reactor. The amount of chlorohydrocarbon moderator is added slowly over a period of several hours until its concentration in the loop is from 0.3 ppmv to 25 ppmv. Suitable clorohydrocarbons used as moderators comprise the C₁ to C₈ chlorohydrocarbons, preferably C₁ to C₄ hydrocarbons and most preferably C₁ to C₂ chlorohydrocarbons. Preferred moderators are ethyl chloride, ethylene dichloride and vinyl chloride, particularly ethyl chloride.

After the chlorohydrocarbon moderator has reached the desired level, oxygen is then added to the recycle feed stream at initially from 5 to 40% of design rate, preferably from 15 to 25% of design rate. Reaction initiation will occur within a few minutes after the addition of the oxygen, after which point the oxygen feed to the reactor, the feed gas to the reactor and the reactor temperature are raised to approximately the design conditions over a period of time ranging from about 15 minutes to about 6 hours.

The catalysts of the instant invention provide for an improved start-up procedure for an ethylene oxide reactor whereby their use allows the reactor to be brought onstream at a higher selectivity to ethylene oxide in a shorter period of time than would be the case if conventional catalysts were used.

The invention will be illustrated by the following illustrative embodiments.

### Illustrative Embodiments

### Illustrative Embodiment 1

The following illustrative embodiment describes typical preparative techniques for making the catalysts of the instant invention (and comparative catalysts) and the typical technique for measuring the properties of these catalysts. Part A: Preparation of stock silver oxalate/ethylene-diamine solution for use in catalyst preparation:
1) Dissolve 415 grams (g) of reagent-grade sodium hydroxide in 2340 millilitres (ml) deionized water. Adjust the temperature to 50 °C.
2) Dissolve 1699 g of "KD" (high purity) silver nitrate in 2100 ml deionized water. Adjust the temperature to 50°C.
3) Add sodium hydroxide solution slowly to silver nitrate solution with stirring while maintaining a temperature of 50°C. Stir for 15 minutes after addition is complete, and then lower the temperature to 40°C.
4) Insert clean filter wands and withdraw as much water as possible from the precipitate created in step (3) in order to remove sodium and nitrate ions. Measure the conductivity of the water removed and add back as much fresh deionized water as was removed by the filter wands. Stir for 15 minutes at 40°C. Repeat this process until the conductivity of the water removed is less than 90 µmho/cm. Then add back 1500 ml deionized water.
5) Add 630 g of high-purity oxalic acid dihydrate in approximately 100 g increments. Keep the temperature at 40°C and stir to mix thoroughly. Add the last portion of oxalic acid dihydrate slowly and monitor pH to ensure that pH does not drop below 7.8.
6) Remove as much water from the mixture as possible using clean filter wands in order to form a highly concentrated silver-containing slurry. Cool the silver oxalate slurry to 30°C.
7) Add 699 g of 92 percent weight (%w) ethylenediamine (8% deionized water). Do not allow the temperature to exceed 30°C during addition.

The above procedure yields a solution containing approximately 27-33%w silver which provides the "stock solution" used in the preparation of Catalysts A through E below.

### Part B: Preparation of impregnation solutions

### For Comparative Catalyst A

129.7 Grams of silver stock solution containing 29.7% Ag was diluted with 14 grams of water and 6.3 grams of monoethanolamine. 0.0285 Grams of NH₄F was dissolved in 2 ml of water and added to the silver solution. CsOH (50% solution in water) in an amount of 0.0582 grams was added to 50 grams of the above diluted silver solution and the resulting mixture was used for the carrier impregnation.

### For Catalyst B

151.7 Grams of stock silver solution containing 30.28% Ag was diluted with 7.5 grams of monethanolamine and 19.8 grams of water. 0.036 Grams of NH₄F and 0.0254 grams of NH₄Cl were dissolved in 2 ml of water and added to the diluted silver solution. A white precipitate of AgCl resulted upon addition, which dissolved upon further stirring. CsOH (50% solution in water) in an amount of 0.0693 grams was added to the above solution and the resulting mixture was used for the carrier impregnation.

### For Catalyst C

151.9 Grams of stock silver solution containing 34.2% Ag was diluted with 27 grams of 50/50 (v/v) mixture of monoethanolamine/H₂O. 0.036 Grams NH₄F and 0.025 grams of NH₄Cl were dissolved in 2 ml water and added to the diluted silver solution. CsOH (50% solution in water) in an amount of 0.0592 grams was added to the above solution and the resulting mixture was used for the carrier impregnation.

### For Comparative Catalyst D

131.2 Grams of stock silver solution containing 29.77% Ag was diluted with 19.8 grams of 50/50 (v/v) mixture of monoethanolamine/H₂O. 0.104 Grams of NaCl were dissolved in 2 ml of water and the resulting solution was added to the diluted silver solution. A white precipitate of AgCl resulted upon addition, but redissolved upon stirring. CsOH (50% solution in water) in an amount of 0.048 grams was added to 50 grams of the above solution and the resulting mixture was used for the carrier impregnation.

### For Catalyst E

151.7 Grams of stock silver solution containing 30.28% Ag was diluted with 7.5 grams of monoethanolamine and 19.8 grams of water. 0.036 Grams of NH₄F and 0.0508 grams NH₄Cl were dissolved in 2 ml water and added to the above diluted silver solution. CsOH (50% solution in water) in an amount of 0.0766 grams was added to 60 grams of the above solution and the resulting mixture was used for the carrier impregnation.

### Part C: Catalyst impregnation and curing

Catalyst carrier D which is described in Table 1 is a preferred support for the instant invention and is used in the following examples and illustrative embodiments unless otherwise stated.

Approximately 30 g of carrier C are placed under 25 mm vacuum for 3 minutes at room temperature. Approximately 50 g of doped impregnating solution is then introduced to submerge the carrier, and the vacuum is maintained at 25 mm for an additional 3 minutes. At the end of this time, the vacuum is released, and excess impregnating solution is removed from the carrier by centrifugation for 2 minutes at 500 rpm. If the impregnating solution is prepared without monoethanolamine, then the impregnated carrier is then cured by being continuously shaken in a 8500 litre/hr. air stream flowing across a cross-sectional area of approximately 3-5 square inches at 250-270°C for 5-6 minutes. If significant monoethanolamine is present in the impregnating solution, then the impregnated carrier is cured by being continuously shaken in a 8500 litre/hr. air stream at 250°C for 2.5 minutes, followed by a 2800 litre/hr. air stream at 270°C for 7.5 minutes (all over a cross-section area of approximately 19.3-32.2 cm²). The cured catalyst is then ready for testing.

This procedure will yield catalysts A through E with the properties show in Table III below.

Catalyst F was prepared using a double impregnation technique. In this technique 120 g of carrier was impregnated with 240 g of silver stock solution with a specific gravity of 1.555. The impregnated carrier was dried/roasted to decompose silver salts to metallic silver. Water pore volume was determined after the first impregnation and was used to calculate the dopant concentrations. 164.7 Grams of stock silver solution with a specific gravity of 1.55 was diluted with 13.9 g of monoethanolamine. 0.1722 Grams of ammonium chloride and 0.0408 g of ammonium fluoride were dissolved in 2.5 g of water and added to the silver solution. 0.0742 Grams of cesium hydroxide (50% solution in water) were added to 60 g of the above silver solution and the resulting mixture was used for the second impregnation of the carrier. The catalyst was cured in a manner similar to that described above. The composition of the resulting catalyst is shown in Table III below.

**TABLE III**

| CATALYST | Ag, wt% | Cs, ppm | Cl, ppm | Cl/Ag Mole Ratio, |
|---|---|---|---|---|
| A | 14.5 | 285 | 0 | 0 |
| B | 15.6 | 273 | 53 | 1.03x10⁻³ |
| C | 15.4 | 283 | 53 | 1.05x10⁻³ |
| D | 14.5 | 257 | 238 | 4.99x10⁻³ |
| E | 14.6 | 306 | 106 | 2.21x10⁻³ |
| F | 27.5 | 260 | 260 | 2.88x10⁻³ |

The actual silver content of the catalyst can be determined by any of a number of standard, published procedures. The actual level of cesium on the catalyst can be determined by employing a stock cesium hydroxide solution, which has been labelled with a radioactive isotope of cesium, in catalyst preparation. The cesium content of the catalyst can then be determined by measuring the radioactivity of the catalyst. Alternatively, the cesium content of the catalyst can be determined by leaching the catalyst with boilinc deionized water. In this extraction process cesium, as well as other alkali metals, is measured by extraction from the catalyst by boiling 10 grams of whole catalyst in 20 millilitres of water for 5 minutes, repeating the above two more times, combining the above extractions and determining the amount of alkali metal present by comparison to standard solutions of reference alkali metals using atomic absorption spectroscopy (using Varian Techtron Model 1200 or equivalent). The actual level of chloride on the catalyst was determined by calculation from the amounts impregnated in the catalyst. The chloride content can also be determined by known analytical techniques such as by fluorescent X-ray analysis.

### Part D: Standard Microreactor Catalyst Test

### Conditions/Procedure

3 to 5 Grams of crushed catalyst of 1.410-0.841 mm (14-20 mesh) are loaded into a 6.4 mm diameter stainless steel U-shaped tube. The U tube is immersed in a molten metal bath (heat medium) and the ends are connected to a gas flow system. The weight of the catalyst used and the inlet gas flow rate are adjusted to achieve a gas hourly space velocity of 3300 ml of gas per ml of catalyst per hour. The inlet gas pressure is 1550 kPa

The gas mixture passed through the catalyst bed (in once-through operation) during the entire test run (including start-up) consists of 30% ethylene, 8.5% oxygen, 5% carbon dioxide, 5 ppmv ethyl chloride with the balance being nitrogen/argon.

The start-up procedure involved ramping the temperature from 180°C to 230°C in the following fashion: 1 hour at 180°C, 1 hour at 190°C, 1 hour at 200°C, 1 hour at 210°C, 1 hour at 220°C, 2 hours at 220°C, 2 hours at 225°C, 2 hours at 230°C, and then the temperature was adjusted to provide an oxygen conversion of 40%. Oxygen conversion (mole %) and selectivity of ethylene to ethylene oxide (mole%) were measured during and after start-up.

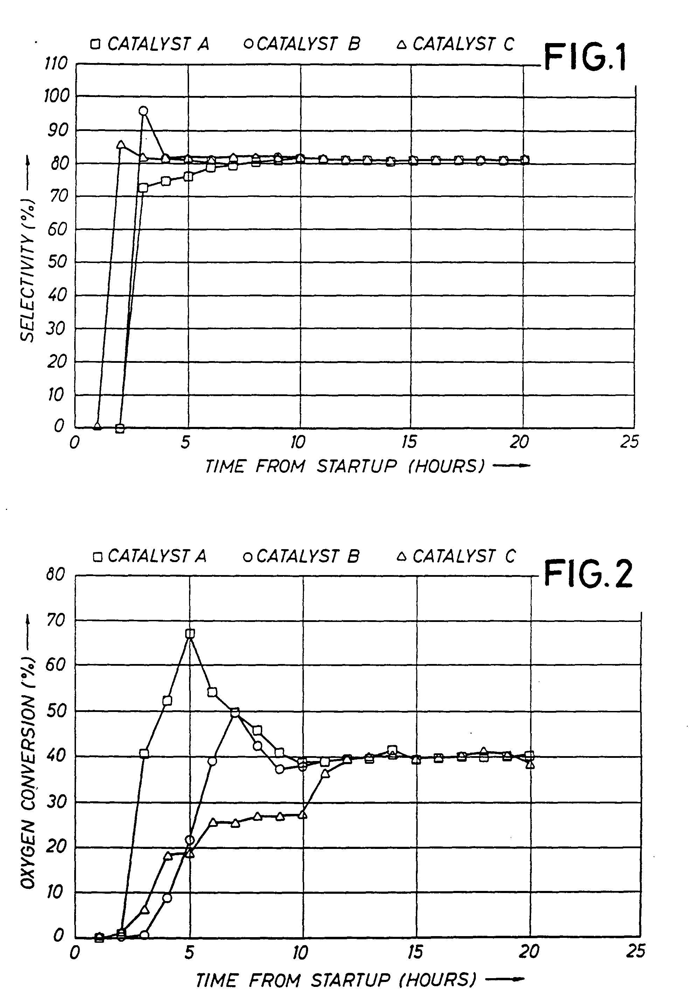

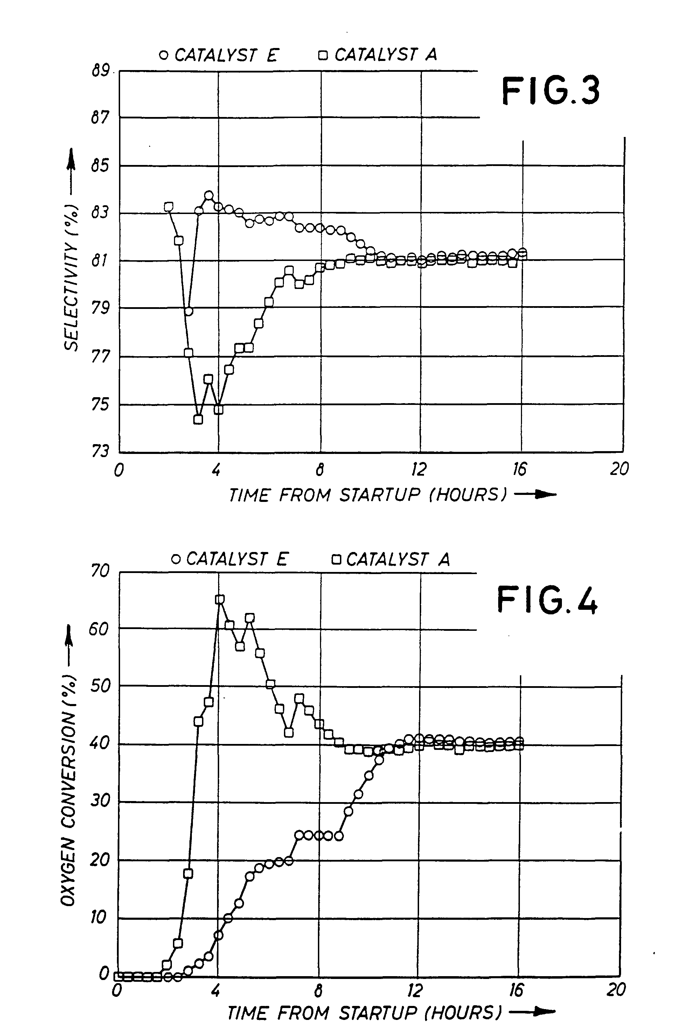

Selectivities and oxygen conversions as a function of time are shown in Figures 1 and 2 for Comparative Catalyst A (no chloride) and chloride-containing Catalysts B and C of this invention and in Figures 3 and 4 for Comparative Catalyst A (no chloride) and Catalyst E of this invention. As can be seen from Figures 1 and 3, the selectivity approaches the final value from the high side for Catalysts B, C and E and from the low side for the Comparative Catalyst A, providing for an earlier enhanced yield of ethylene oxide with the use of the chloride-containing catalysts. From Figures 2 and 4 it can be seen that the Comparative Catalyst A shows a very high oxygen conversion approaching 70%, which provides for a very "hot" start-up which is much more difficult to control than the start-up using the catalysts of this invention.

For Comparative Catalyst D it was found that after start-up the catalyst had suffered a significant selectivity loss (decrease of 3.4%) and activity loss (increase of 35°C) when compared to a catalyst without chloride (Comparative Catalyst A). Catalyst F did not show any loss in selectivity or activity.

## Claims

1. A catalyst for the vapour phase production of ethylene oxide from ethylene and oxygen in the presence of a hydrocarbon moderator comprising, expressed as the metal by weight of the total catalyst, from 12 to 40 percent of silver and from 10 to 3000 parts per million of an alkali metal at least 50% of which is selected from potassium, rubidium, cesium, lithium and mixtures thereof, and further comprising prior to the first use of the catalyst from 0.4x10⁻³ to 4x10⁻³ moles of chloride per mole of silver, deposited by impregnation followed by curing without the use of superheated steam on a carrier comprising at least 85 percent by weight of alpha alumina.

2. The catalyst of claim 1 wherein the carrier has a water pore volume between 0.1 and 0.6 ml/g and surface area between 0.1 and 3 m²/g.

3. The catalyst of claim 1 or 2, wherein the silver ranges from 12 to 18 weight percent of the total catalyst and the chloride content ranges from 20 to 200 parts per million by weight of the total catalyst.

4. The catalyst of any one of claims 1-3, wherein one or more additional promoters or copromoters are present, selected from rhenium, sulphur, molybdenum, tungsten and chromium.

5. A process for preparing a catalyst for the vapour phase production of ethylene oxide from ethylene and molecular oxygen which comprises impregnating a carrier comprising at least 85 percent by weight of alpha-alumina with one or more solutions comprising solvent having silver compound(s) dissolved therein, compound(s) of alkali metal(s) selected from potassium, rubidium, cesium, lithium and mixtures thereof dissolved therein and chloride-containing compounds dissolved therein sufficient to deposit on the support from 12 to 40 percent by weight of the total catalyst of the silver compound(s), expressed as the metal, from 10 to 3000 parts per million by weight of the alkali metal compound(s), at least 50% of which is selected from potassium, rubidium, cesium, lithium and mixtures thereof, expressed as the metal, by weight of the total catalyst and from 0.4x10⁻³ to 4x10⁻³ moles of chloride per mole of silver deposited and curing without the use of superheated steam.

6. The process of claim 5, wherein after impregnation, the silver is reduced to metallic silver by heating at a temperature between 50 °C to 600 °C.

7. The process of claim 5 or 6, wherein the chloride-containing compound is an alkali metal chloride or ammonium chloride.

8. A process of starting up an ethylene oxide reactor containing fresh catalyst by heating the catalyst to ethylene oxide forming temperatures in the presence of ethylene, oxygen and a chlorohydrocarbon moderator, characterized in that the fresh catalyst is according to any one of claims 1-4.

## Patentansprüche

1. Katalysator für die Dampfphasenproduktion von Ethylenoxid aus Ethylen und Sauerstoff in Gegenwart eines Kohlenwasserstoffmoderators, welcher Katalysator, ausgedrückt als Metall und bezogen auf das Gewicht des Gesamtkatalysators, von 12 bis 40% Silber und von 10 bis 3.000 Teile pro Million eines Alkalimetalles, von dem wenigstens 50% unter Kalium, Rubidium, Cäsium, Lithium und deren Gemischen ausgewählt sind, umfaßt und der zusätzlich vor der ersten Anwendung des Katalysators von 0,4x10⁻³ bis 4x10⁻³ Mol Chlorid pro Mol Silber umfaßt, abgelagert durch Imprägnieren mit anschließendem Härten ohne Anwendung von überhitztem Dampf auf einem Träger, der wenigstens 85 Gew.-% α-Aluminiumoxid umfaßt.

2. Katalysator nach Anspruch 1, worin der Träger ein Wasserporenvolumen zwischen 0,1 und 0,6 ml/g und eine Oberflächengröße zwischen 0,1 und 3 m²/g aufweist.

3. Katalysator nach Anspruch 1 oder 2, worin der Silbergehalt von 12 bis 18 Gew.-% des Gesamtkatalysators ausmacht und der Chloridgehalt in einem Bereich von 20 bis 200 Teilen pro Million, bezogen auf das Gewicht des Gesamtkatalysators, liegt.

4. Katalysator nach einem der Ansprüche 1 bis 3, worin ein oder mehrere weitere Promotoren oder Copromotoren zugegen sind, ausgewählt unter Rhenium, Schwefel, Molybdän, Wolfram und Chrom.

5. Verfahren zur Herstellung eines Katalysators für die Dampfphasenproduktion von Ethylenoxid aus Ethylen und molekularem Sauerstoff, welches Verfahren ein Imprägnieren eines Trägers, der wenigstens 85 Gew.-% α-Aluminiumoxid umfaßt, mit einer oder mit mehreren Lösungen mit einem Gehalt an Lösungsmittel und mit darin gelöster Silberverbindung bzw. gelösten Silberverbindungen, einer oder mehreren, darin gelösten Verbindungen eines oder mehrerer Alkalimetalle, ausgewählt unter Kalium, Rubidium, Cäsium, Lithium und deren Gemischen, und darin gelösten chloridhältiger Verbindungen, in ausreichender Menge, um auf dem Träger 12 bis 40 Gew.-% der Silberverbindung(en), ausgedrückt als das Metall und bezogen auf den Gesamtkatalysator, von 10 bis 3.000 Teile pro Million Alkalimetallverbindung(en), ausgedrückt als das Metall und bezogen auf das Gewicht des Gesamtkatalysators, und von 0,4x10⁻³ bis 4x10⁻³ Mol Chlorid pro Mol abgelagertem Silber abzulagern, und ein Härten ohne Anwendung von überhitztem Dampf umfaßt.

6. Verfahren nach Anspruch 5, worin nach dem Imprägnieren das Silber durch Erhitzen auf eine Temperatur zwischen 50°C und 600°C zu metallischem Silber reduziert wird.

7. Verfahren nach Anspruch 5 oder 6, worin die chloridhältige Verbindung ein Alkalimetallchlorid oder Ammoniumchlorid ist.

8. Verfahren zum Anfahren eines frischen Katalysator enthaltenden Ethylenoxidreaktors durch Erhitzen des Katalysators auf Ethylenoxidbildungstemperaturen in Gegenwart von Ethylen, Sauerstoff und eines Chlorkohlenwasserstoffmoderators, dadurch gekennzeichnet, daß der frische Katalysator ein solcher gemäß einem der Ansprüche 1 bis 4 ist.

## Revendications

1. Catalyseur pour la production en phase vapeur d'oxyde d'éthylène à partir d'éthylène et d'oxygène en présence d'un modérateur hydrocarboné comprenant, exprimés en tant que métal en poids du catalyseur total, de 12 à 40% d'argent et de 10 à 3000 parties par million d'un métal alcalin dont au moins 50% sont choisis parmi le potassium, le rubidium, le césium, le lithium et leurs mélanges, et comprenant de plus avant la première utilisation du catalyseur de 0,4x10⁻³ à 4x10⁻³ mole de chlorure par mole d'argent, déposé par imprégnation suivie d'une cuisson sans l'utilisation de vapeur surchauffée sur un support comprenant au moins 85% en poids d'alpha-alumine.

2. Catalyseur suivant la revendication 1, dans lequel le support a un volume des pores dans l'eau entre 0,1 et 0,6 ml/g et une aire superficielle entre 0,1 et 3 m²/g.

3. Catalyseur suivant l'une et l'autre des revendications 1 et 2, dans lequel l'argent constitue de 12 à 18% en poids du catalyseur total et la teneur en chlorure constitue de 20 à 200 parties par million en poids du catalyseur total.

4. Catalyseur suivant l'une quelconque des revendications 1 à 3, dans lequel un ou plusieurs promoteurs ou copromoteurs additionnels sont présents, choisis parmi le rhénium, le soufre, le molybdène, le tungstène et le chrome.

5. Procédé de préparation d'un catalyseur pour la production en phase vapeur d'oxyde d'éthylène à partir d'éthylène et d'oxygène moléculaire, qui comprend l'imprégnation d'un support comprenant au moins 85% en poids d'alpha-alumine avec une ou plusieurs solutions comprenant un solvant dans lesquelles sont dissous un ou des composés d'argent, sont dissous un ou des composés de métal alcalin ou de métaux alcalins choisis parmi le potassium, le rubidium, le césium, le lithium et leurs mélanges et sont dissous des composés contenant du chlorure en quantité suffisante pour déposer sur le support de 12 à 40% en poids du catalyseur total du ou des composés d'argent, exprimés en tant que métal, de 10 à 3000 parties par million en poids du ou des composés de métal alcalin, dont au moins 50% sont choisis parmi le potassium, le rubidium, le césium, le lithium et leurs mélanges, exprimés en tant que métal, en poids du catalyseur total et de 0,4x10⁻³ à 4x10⁻³ mole de chlorure par mole d'argent déposé et la cuisson sans l'utilisation de vapeur surchauffée.

6. Procédé suivant la revendication 5, dans lequel après imprégnation, l'argent est réduit en argent métallique en chauffant à une température de 50°C à 600°C.

7. Procédé suivant l'une ou l'autre des revendications 5 et 6, dans lequel le composé contenant du chlorure est un chlorure de métal alcalin ou le chlorure d'ammonium.

8. Procédé de démarrage d'un réacteur à oxyde d'éthylène contenant du catalyseur frais en chauffant le catalyseur à des températures de formation d'oxyde d'éthylène en présence d'éthylène, d'oxygène et d'un modérateur de chlorohydrocarbure, caractérisé en ce que le catalyseur frais est suivant l'une quelconque des revendications 1 à 4.
